(19) Europäisches Patentamt
European Patent Office
Office européen des brevets

(11) Publication number: 0 664 129 A2

# (12) EUROPEAN PATENT APPLICATION

(21) Application number: 95100081.9

(51) Int. Cl.$^{6}$: A61K 31/575

(22) Date of filing: 04.01.95

(30) Priority: 25.01.94 GB 9401402

(43) Date of publication of application:
26.07.95 Bulletin 95/30

(84) Designated Contracting States:
DE GB IT

(71) Applicant: PHARMACIA S.p.A.
Via Robert Koch, 1.2
I-20152 Milano (IT)

(72) Inventor: Ghiselli, Giancarlo
Via Cardinal Tosi 5
I-21052 Busto Arsizio,
(Varese) (IT)
Inventor: Lovisolo, PierPaolo
Via Vigevano 43
I-20144 Milan (IT)
Inventor: Chiari, Augusto
Piazza San Jacopino 7
I-50100 Florence (IT)
Inventor: Baratte', Simona
Via Pisacane 9
I-20010 Cornaredo,
(Milan) (IT)
Inventor: Fancelli, Daniele
Via Gianella 21
I-20152 Milan (IT)
Inventor: Severino, Dino
Via A. Magnasco 6
I-20149 Milan (IT)

(74) Representative: Ferrario, Vittorino
PHARMACIA S.p.A.,
Patent and Documentation Dept.,
Via Bisceglie, 104
I-20152 Milano (IT)

## (54) Epicholesterol for treating dyslipidemia.

(57) New pharmaceutical composition comprising a pharmaceutically acceptable carrier and/or diluent and, as an active principle, epicholesterol, having in particular cholesterol absorption inhibiting activity and/or blood cholesterol lowering activity.

EP 0 664 129 A2

The present invention relates to the use of epicholesterol in lowering abnormal levels of blood and tissue lipids in mammals.

As known hyperlipidemia is a condition in which elevated concentrations of triglycerides or cholesterol are detected in fasting plasma. Clinical concern arises because elevated concentrations of plasma lipids, in particular cholesterol, are an established risk factor in the development of atherosclerosis; in addition. therapeutic agents which control the level of serum cholesterol have proven to be effective in the treatment of coronary artery disease.

While agents exist that can modulate circulating levels of cholesterol carrying lipoproteins, these agents have little or no effect on the intestinal absorption of cholesterol. Dietary cholesterol can increase the level of serum cholesterol to levels which place an individual at increased risk for the development or exacerbation of atherosclerosis.

Therefore there is a need in therapy of agents capable of lowering abnormal levels of lipids, in particular cholesterol, in mammals including humans.

Now we have found that epicholesterol has a potent inhibitory activity on the absorption of dietary cholesterol and is a potent agent in lowering the concentration of blood and tissue cholesterol in mammals.

Accordingly, object of the present invention is to provide a pharmaceutical composition comprising a pharmaceutically acceptable carrier and/or diluent and, as an active principle, epicholesterol, having in particular cholesterol absorption inhibiting activity and/or blood cholesterol lowering activity.

Object of this invention is also a composition for lowering, blood- and/or tissue-cholesterol and/or treating hyperlipoproteinemias in mammals comprising in combination a material selected from the group consisting of food and a pharmaceutically acceptable carrier, in particular for oral administration, and epicholesterol.

The present invention provides also the use of epicholesterol in the preparation of a medicament for use as cholesterol absorption inhibiting agent and/or cholesterol lowering agent.

The term "epicholesterol" as used herein means the commercially available cholest-5-en-3α-ol, i.e. the compound having the following formula

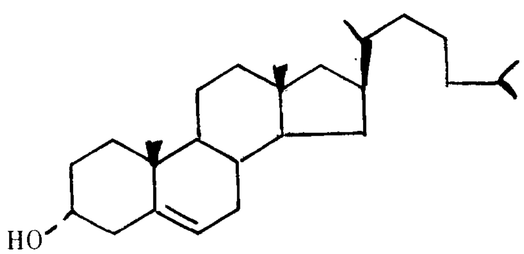

A method for epicholesterol preparation is decribed, for instance, by Corey et al., in Tetrahedron Letters, (1975), 37, 3183-3186.

Individuals suffering from hyperlipoproteinemias should consume a diet that minimizes concentrations of lipids in plasma.

Therefore they should be placed, in particular, on a diet that is low in cholesterol and in fats, in general.

Now we have found that adding epicholesterol to a hypercholesterolemic diet results in preventing the expected effect of the diet on plasma lipids level.

Accordingly, object of this invention is also the use of epicholesterol in the preparation of a medicament for use in the treatment of hyperlipoproteinemias.

Epicholesterol is herein also defined as the "compound of the invention" and as the "active principle of this invention".

In view of its high biological activity, epicholesterol can be used in medicine or added to the diet consumed by mammals, including humans.

In accordance with the present invention, epicholesterol can be administered to mammals, including humans, preferably orally. The therapeutic regimen must be adapted to the type of pathology, taking into account, as usual, the route of administration, the form in which the compound is administered and the age, weight and conditions of the subject involved. The oral route is employed, in general, for all the conditions requiring such compound. The dosage for oral administration of epicholesterol to an average 70 kilo individual will be from about 100 mg to about 5 g per day, preferably from about 250 mg to about 1 g per day.

The nature of the pharmaceutical compositions containing the compound of this invention in association with pharmaceutically acceptable carriers or diluents will, of course, depend upon the desired route of administration.

The compositions may be formulated in the conventional manner with the usual ingredients. For example, the compound of the invention may be administered in the form of oily solutions or aqueous or oily suspensions, tablets, pills, gelatine capsules, syrups or drops.

Thus, for oral administration, the pharmaceutical compositions, containing the compound of this invention, are preferably tablets, pills or gelatine capsules which contain the active substance together with diluents, such as lactose, dextrose, sucrose, mannitol, sorbitol, cellulose; lubricants, for instance silica, talc, stearic acid, magnesium or calcium stearate, and/or polyethylene glycols; or they may also contain binders, such as starches, gelatine, methylcellulose, carboxymethylcellulose, gum-arabic, tragacanth, polyvinylpyrrolidone; disaggregating agents, such as starches, alginic acid, alginates, sodium starch glycolate; dyestuffs; sweeteners; wetting agents, such as lecithin, polysorbates, laurylsulphates and in general, non-toxic and pharmacologically inactive substances used in pharmaceutical formulations.

Said pharmaceutical preparations may be manufactured in known manner, for example by means of mixing, granulating, tabletting, sugar-coating, or film-coating processes.

The liquid dispersions for oral administration may be e.g. syrups, emulsions and suspensions.

The syrups may contain as carrier, for example, saccharose with glycerine and/or mannitol and/or sorbitol.

The suspensions and the emulsions may contain as carrier, for example, a natural gum, agar, sodium alginate, pectin, methylcellulose, carboxymethylcellulose, or polyvinyl alcohol.

If desired, epicholesterol can be administered in a finally dispersed powder which is typically mixed with food diet.

When combined with the diet, epicholesterol can be used for instance in an amount up to about 1% by weight thereof. Epicholesterol can be dissolved in a suitable pharmaceutically acceptable solvent, such as acetone, which is then mixed with food and therafter the solvent is evaporated to leave epicholesterol in finely dispersed powdered form thoroughly mixed throughout the food.

A typical capsule which can be prepared according to this invention will contain 100 mg epicholesterol, 50 mg lactose, 50 mg dicalcium phosphate, 2 mg magnesium stearate and 10 mg of talc. Typical tablets can contain 250 mg epicholesterol, 7 mg magnesium stearate, 228 mg of dicalcium phosphate bihydrate, 228 mg D.C. lactose, 36 mg sodium starch glycolate and 1 mg silicon dioxide.

## PHARMACOLOGY

### Animals

Male Sprague Dawley rats weighing 250 g and male CD mice weighing 22-25 g (Charles River) were housed in rooms maintained at 21±1 °C with relative humidity 50-60% and a 12 hours light-dark cycle. After a week of housing, the animals were allocated to the different experimental groups and received one of the following diets:

a) rat or mouse chow diet, containing 0.5% sodium cholate;
b) cholesterol enriched diet, containing 0.5% sodium cholate and 1.5% cholesterol;
c) epicholesterol enriched diet, containing 0.5% sodium cholate and 1.5% epicholesterol;
d) epicholesterol and cholesterol enriched diet containing 0.5% sodium cholate and 1.5% of both sterols.

Weight of the animals and food consumption were recorded daily.

### Lipid Extraction and Determination

After overnight fasting, the animals were anesthetized with sodium pentobarbital (50 mg/kg body weight) and exsanguinated from the abdominal aorta. Plasma was obtained by low speed (3000 g for 10 min) centrifugation of whole blood containing $Na_2$EDTA as anticoagulant.

Plasma lipid extracts were prepared according to Bligh E.G., Dyer W.J., Can. J. Bioch. Physiol, 31, 911, 1959.

Liver specimens (500 mg) were excised from the lateral liver lobe, washed in ice-cold saline, and immediately placed on dry-ice and stored at -80 °C. They were extracted according to the method of Folch J. et al., J. Biol. Chem. , 226, 497, 1957, as modified by Carlson L.A., J. Atheroscler. Res., 3, 334, 1963.

Plasma and liver total cholesterol content (i.e. unesterified and esterified cholesterol fractions) were determined by the enzymatic colorimetric method of Allain et al., Clin. Chem., 20, 470, 1974. In this assay

epicholesterol is not read.

Gas Liquid Chromatography of Sterols

Lipids extract from plasma and liver were separated by TLC on Silica Gel plates from Merck using petroleum ether 40-70°/diethyl ether/acetic acid (80:20:1) as developing system. Cholesterol, epicholesterol, dihydrocholesterol, and 7-keto-cholesterol as representative of the oxysterol class of sterols migrated with very similar Rf and could be recovered as a single area from the TLC plate. These sterols were eluted from the silica using chloroform and were dried by evaporation under $N_2$. The samples were redissolved in pyridine and derivatizated adding an equal volume of MBSTFA [N-methyl-N(tert-butyldimethylsilyl)-Trifluoroacetamide].

After 20 minutes at room temperature the samples were analyzed by GLC according to Smith N.B., Lipids, 17, 464, 1982, on a Carlo Erba Mega 3000 gaschromatographer equipped with a flame ionization detector and a 25 m OV1 column (0.2 mm id., film thickness 0.2-0.25). He was used as carrier at 100 Kps. The splitless injection technique was used and 1-2 µl sample were injected. The oven temperature, initially at 130°C, was rapidly increased (30°C/min) to 285°C, the temperature was increased thereafter at 1.5°C/min to 330°C. The injection and the detector temperatures were respectively 340°C and 350°C. Retention time of cholesterol, epicholesterol, dihydrocholesterol and 7keto-cholesterol were determined by injecting pure sterols.

Micellar Cholesterol Solubilization

The micellar solubility of cholesterol in vitro was measured by the method of Ikeda I., Tanabe Y. and Sugano M. , J. Nutr. Sci. Vitaminol. 35, 361, 1989. In detail, cholesterol (1 mM) or mixture of cholesterol (1 mM) and epicholesterol (0.5-8 mM) were sonicated for 2 minutes at 37°C in 15 mM $NaPO_4$ buffer, pH 7.4, containing 0.6 mM egg yolk lecithin, 1 mM oleic acid, 0.5 mM monolein, 6.6 mM Na taurocholate, and 132 mM NaCl. After incubation at 37°C overnight, the solutions were subjected to ultracentrifugation (100,000 g for 60 min at 37°C).

Samples of the supernatant were assayed for the cholesterol content.

RESULTS AND DISCUSSION

Results of the in vivo experiments are illustrated in Table I. Both in rats and mice, fed 4 days with a cholesterol-enriched diet, plasma total cholesterol rose significantly and at least a fourfold increase in total cholesterol content occurred in the liver. Supplementing the diet with the same amount as cholesterol of epicholesterol, brought about a significant decrease of the cholesterol-enriched diet induced hypercholesterolemia and of cholesterol accumulation in the liver.

Noteworthy, feeding rats and mice with a diet enriched with epicholesterol alone did not brought about any significant change in the cholesterol content of plasma and liver compared to the control groups.

TABLE I

**EFFECT OF CHOLESTEROL AND OF EPICHOLESTEROL ON THE CHOLESTEROL CONCENTRATION IN PLASMA (mg/dl) AND LIVER (mg/g fresh tissue) OF RATS AND MICE**

| Dietary Groups | Rats | | Mice | |
|---|---|---|---|---|
| | Plasma | Liver | Plasma | Liver |
| Controls | 64±12 (n=10) | 2.140±18 (n=10) | 109±16 (n=10) | 2.58±18 (n=10) |
| 1.5% Cholesterol | **178±45 (n=7) | **11.28±1.48 (n=7) | **152±19 (n=10) | **18.70±2.60 (n=10) |
| 1.5% Epicholesterol | °° 64±18 (n=3) | °° 2.59±0.38 (n=3) | * 93±15 °°(n=10) | °° 2.58±0.16 (n=10) |
| 1.5% Cholesterol 1.5% Epicholesterol | **103±9 ° (n=3) □ | ** 5.71±0.95 °° (n=3) □ | **115±26 □(n=10) | ** 9.54±1.49 °° (n=10) □□ |

* p<0.05 vs Control Group - ** p<0.01 vs Control Group
° p<0.05 vs Cholesterol Group - °° p<0.01 vs Cholesterol Group
□ p<0.05 vs Epicholesterol Group - □□ p<0.01 vs Epicholesterol Group

In order to further investigate the effect of epicholesterol feeding on the plasma and liver sterol contents, the relative content of cholesterol and epicholesterol was assessed by GLC in the plasma and the liver of mice fed either a control diet or a 1.5% cholesterol-enriched diet, or finally 1.5% epicholesterol-enriched diet. Results are presented in Table II. Inclusion of epicholesterol in the diet lead to the appearance of only trace amount of this sterol in the plasma and the liver.

TABLE II

**EFFECT OF CHOLESTEROL AND OF EPICHOLESTEROL ENRICHED DIETS ON THE MICE PLASMA AND LIVER**

**UNESTERIFIED STEROL CONTENT (Mean ± SD, n=3)**

| | Dietary Groups | Percent Distribution | | | |
|---|---|---|---|---|---|
| | | Cholesterol | Epicholesterol | Dihydrocholesterol | Oxysterols |
| **Plasma** | Controls | 97.6±2.5 | nd* | nd | 2.4±2.5 |
| | 1.5% Cholesterol | 97.4±0.8 | nd | nd | 3.3±1.9 |
| | 1.5% Epicholesterol | 87.4±1.0 | 2.6±1.5 | 8.5±0.9 | 1.5±1.3 |
| **Liver** | Controls | 76.7±3.1 | nd | nd | 23.3±3.0 |
| | 1.5% Cholesterol | 83.6±7.7 | nd | nd | 16.4±7.7 |
| | 1.5% Epicholesterol | 75.7±4.5 | 1.0±0.4 | 9.0±1.5 | 14.3±5.8 |

* nd: not detectable

In order to investigate the mechanism of action of the hypocholesterolemic effect of epicholesterol, we assessed the effect of epicholesterol on the micellar solubility of cholosterol. Results of experiments are presented in Table III. When epicholesterol and cholesterol were mixed together, the cholesterol micellar solubility was significantly decreased at all the concentrations tested of epicholesterol. For example by mixing cholesterol and epicholesterol in a 1 to 0.5 molar ratio, we found that the cholesterol micellar

solubility can decrease by more than 30%. Increasing the epicholesterol to cholesterol ratio, further decreased the cholesterol micellar solubility.

Altogether these results are consistent with our idea that epicholesterol decreases the micellar solubility of cholesterol, an important determinant of the rate of cholesterol absorption in the gut. On the other hand, epicholesterol is only minimally absorbed by the intestine, and enters the blood stream and accumulates in the liver to a very small extent.

TABLE III

| MICELLAR CHOLESTEROL SOLUBILITY: EFFECT OF EPICHOLESTEROL | |
|---|---|
| Cholesterol-to-Epicholesterol Ratio (mM) | Micellar Cholesterol Content (mM) |
| Control (Cholesterol Only) | 1.00 ± 0.05 |
| 2* | 0.71 ± 0.05 |
| 1 | 0.42 ± 0.03 |
| 0.5 | 0.23 ± 0.06 |
| 0.25 | 0.22 ± 0.03 |
| 0.125 | 0.18 ± 0.02 |

* When epicholesterol was present all the values are significantly ($p<0.0001$) different from the control values.

## Claims

**1.** A pharmaceutical composition comprising a pharmaceutically acceptable carrier and/or diluent and, as an active principle,epicholesterol.

**2.** A pharmaceutical composition, according to claim 1, having cholesterol absorption inhibiting activity and/or cholesterol blood lowering activity.

**3.** A composition for lowering blood- and/or tissue-cholesterol and/or treating hyperlipoproteinemias comprising in combination a material selected from the group consisting of food and a pharmaceutically acceptable carrier, and epicholesterol.

**4.** The use of epicholesterol in the preparation of a medicament for use as cholesterol absorption inhibiting agent and/or cholesterol lowering agent.

**5.** The use of epicholesterol in the preparation of a medicament for use in the treatment of hyperlipoproteinemias.